# EUROPEAN PATENT APPLICATION

(11) **EP 4 807 013 A1**
(43) Date of publication of application: **16.09.2026**
(21) Application number: 23958155.6
(22) Date of filing: 19.12.2023
(51) Int. Cl.: C12P 7/64, C12R 1/74, C12R 1/72

(54) **METHOD FOR PRODUCING LONG-CHAIN DICARBOXYLIC ACID, AND PRODUCT**

(30) Priority: 08.11.2023 CN 202311479503
(71) Applicant: Cathay Biotech Inc., Shanghai 201203 (CN); Cibt America Inc., Newark DE 19713 (US)
(72) Inventor: HAO, Yingli, Shanghai 201203 (CN); BAO, Jiawei, Shanghai 201203 (CN); XU, Min, Shanghai 201203 (CN); LIU, Xiucai, Shanghai 201203 (CN)
(74) Representative: SSM Sandmair
(86) International application number: PCT/CN2023/139787
(87) International publication number: WO 2025/097557

(57) **Abstract**

Provided are a method for producing a long-chain dicarboxylic acid and a long-chain dicarboxylic acid product. The method comprises: inoculating a seed liquid of a fermentation strain into a fermentation tank, where a fermentation substrate comprises any one or more among a fatty acid, a derivative thereof, and an alkane. Eco-friendly raw materials such as a fatty acid, a derivative thereof, and a plant-based alkane are used as substrates, which partially or completely replace fossil fuels such as petroleum alkanes; the product is an eco-friendly long-chain dicarboxylic acid, bio-based contents can be controlled, and the fermentation method retains a relatively high yield and conversion rate.

## Description

### TECHNICAL FIELD

The present disclosure pertains to the technical field of fermentation, and particularly relates to a method for producing a long-chain dicarboxylic acid and a long-chain dicarboxylic acid product.

### BACKGROUND

Long-chain dicarboxylic acids have widespread applications in multiple fields due to the uniqueness of their molecular structures and reactivity. Specialty nylons (such as polyamides), high-grade fragrances, high-end hot melt adhesives, cold-resistant plasticizers, high-grade lubricants, high-grade rust inhibitors, high-grade paints and coatings, and the like can be synthesized using the long-chain dicarboxylic acids as raw materials.

Long-chain dicarboxylic acids have important and widespread industrial uses, but they do not exist independently in nature, and their sources vary depending on the chain length. Synthesis methods include chemical synthesis methods and biological fermentation methods. Chemical synthesis methods are characterized by complex processes, harsh conditions, many steps, low yields, low purity, high costs, pollution, and the like. Biological methods typically use corresponding long-chain alkanes and derivatives thereof as substrates to produce long-chain dicarboxylic acids by fermentation using *Candida tropicalis*. Compared with chemical synthesis methods, biological methods are characterized by simple production processes, mild conditions, specific reactions, higher yields, low energy consumption, and simple extraction and purification processes.

Research work on long-chain dicarboxylic acids has been conducted for more than 40 years in China, mainly using petroleum-based alkanes as substrates. Novel plant-based resources such as fatty acids, fatty acid salts, and fatty acid esters can replace fossil raw materials such as petroleum-based alkanes, which is of great significance for the sustainable development of industrial processes.

### SUMMARY OF THE INVENTION

The present disclosure provides a method for producing a long-chain dicarboxylic acid and a product. The method can use eco-friendly fermentation substrates, i.e., fatty acids and derivatives thereof such as fatty acid salts and fatty acid esters, as well as plant-based alkanes, to partially or completely replace fossil fuels such as petroleum-based alkanes, and can maintain relatively high yield and conversion rate.

To achieve the above objective, in a first aspect, the present disclosure is to provide a method for producing a long-chain dicarboxylic acid, and the method comprises: inoculating a seed liquid of a fermentation strain into a fermentation tank, where a fermentation substrate comprises any one or more of a fatty acid and a derivative thereof, and an alkane.

In some embodiments, the long-chain dicarboxylic acid has a chemical formula of HOOC(CH₂)nCOOH, wherein n ≥ 8; the long-chain dicarboxylic acid comprises: any one of decanedioic acid, undecanedioic acid, dodecanedioic acid, tridecanedioic acid, tetradecanedioic acid, pentadecanedioic acid, hexadecanedioic acid, heptadecanedioic acid, and octadecanedioic acid.

In some embodiments, the fatty acid derivative comprises a fatty acid ester and a fatty acid salt.

In some embodiments, the fermentation substrate comprises any one or a combination of two or more of a fatty acid, a fatty acid ester, a fatty acid salt, and an alkane.

In some embodiments, the fermentation substrate comprises at least one of a fatty acid, a fatty acid ester, and a fatty acid salt.

In some embodiments, the fermentation substrate comprises an alkane and at least one of a fatty acid, a fatty acid ester, and a fatty acid salt.

In some embodiments, the fermentation substrate is a fatty acid and an alkane.

In some embodiments, the fatty acid and the alkane have the same number of carbon atoms.

In some embodiments, the alkane is a petroleum-based alkane and/or a bio-based alkane.

In some embodiments, the fatty acid is a linear monocarboxylic acid having 10 or more carbon atoms, preferably a linear monocarboxylic acid having 10-18 carbon atoms, comprising: any one of decanoic acid, undecanoic acid, lauric acid (alias: dodecanoic acid), tridecanoic acid, myristic acid, pentadecanoic acid, palmitic acid (alias: hexadecanoic acid), heptadecanoic acid, and octadecanoic acid.

In some embodiments, the fatty acid salt comprises any one of a sodium salt, a potassium salt, an ammonium salt, and a calcium salt of a fatty acid. The fatty acid has the same definition as above. Fatty acids mainly come from the seeds of oil-rich tropical crops such as palm trees and coconut trees. This novel renewable resource can replace fossil raw materials such as petroleum alkanes, which is of great significance for the sustainable development of industrial processes.

In some embodiments, the fatty acid ester comprises any one of a decanoate, an undecanoate, a laurate, a tridecanoate, a myristate, a pentadecanoate, a palmitate, a heptadecanoate, and an octadecanoate, such as methyl laurate, ethyl laurate, butyl laurate, methyl myristate, ethyl myristate, butyl myristate, methyl palmitate, ethyl palmitate, and butyl palmitate. Since most fatty acid esters are liquid at room temperature, have a good dispersion effect, and have characteristics similar to those of alkanes, they can replace the substrate alkanes used at the present stage. Fatty acid esters mainly come from the seeds of oil-rich tropical crops such as palm trees and coconut trees. This novel renewable resource can replace fossil raw materials such as petroleum alkanes, which is of great significance for the sustainable development of industrial processes. The fatty acid has the same definition as above.

In some embodiments, the alkane comprises a linear alkane having 10 or more carbon atoms, preferably a linear alkane having 10-18 carbon atoms, comprising: any one of decane, n-undecane, n-dodecane, n-tridecane, n-tetradecane, n-pentadecane, n-hexadecane, n-heptadecane, and n-octadecane.

In some embodiments, the fermentation substrate is a fatty acid and an alkane; the fatty acid and the alkane are in a mass ratio of (0.1-25):15, preferably (1-20):15.

In some embodiments, the fermentation strain comprises *Candida viswanathii*, *Candida albicans*, *Candida tropicalis*, *Candida sake*, or *Yarrowia lipolytica*.

In some embodiments, the seed liquid is inoculated into a fermentation tank at an inoculation amount of 10%-50%. The percentages represent volume percentages (v/v).

The fermentation tank contains a fermentation culture medium. The fermentation culture medium comprises a nitrogen source, a carbon source, a growth factor, and an inorganic salt, and may further comprise an additive such as a defoamer.

In some embodiments, the fermentation culture medium comprises the following components: 0%-1.0% of corn steep liquor, 2.0%-6.0% of glucose, 0%-0.8% of yeast extract, 0.4%-1.0% of potassium dihydrogen phosphate, 0.2%-0.6% of magnesium sulfate, 0.1%-1% of ammonium sulfate, and 0.2%-0.8% of potassium nitrate.

In some embodiments, the seed liquid of the fermentation strain is inoculated into a fermentation tank containing a fermentation culture medium and cultured.

In some embodiments, the seed liquid of the fermentation strain has an optical density value OD₆₂₀ of 0.5-1.0 after a 30-fold dilution.

In some embodiments, a method for preparing the seed liquid comprises the following steps: subjecting the fermentation strain to an activation culture in a shake flask, and when the shake flask seed has an optical density value OD₆₂₀ of 0.5-1.0 after a 30-fold dilution, inoculating the shake flask seed into a seed tank containing a seed culture medium for seed culture until the obtained seed liquid has an optical density value OD₆₂₀ of 0.5-1.0 after a 30-fold dilution.

Conditions for the activation culture comprise: a temperature of 27-35°C, a shaker rotation speed of 150-250 rpm, and a time of 38-42 h.

Conditions for the seed culture comprise: a temperature of 27-35°C, an aeration rate of 0.2-0.8 vvm, a pressure of 0.05-0.20 MPa, and an amount of dissolved oxygen of 5%-50%.

The seed culture medium comprises a nitrogen source, a carbon source, an inorganic salt, a growth factor, a defoamer, and the like; types of the carbon source of the seed culture medium include glucose, maltose, sucrose, and the like; types of the nitrogen source include corn steep liquor, urea, yeast extract, potassium nitrate, ammonium sulfate, and the like; types of the inorganic salt include potassium dihydrogen phosphate, dipotassium hydrogen phosphate, sodium dihydrogen phosphate, potassium nitrate, sodium chloride, ferrous sulfate, magnesium sulfate, and the like; types of the nutrition factor include vitamin B1, vitamin B2, vitamin B5, biotin, and the like.

In some embodiments, the seed culture medium comprises the following components: 0.2%-0.8% of corn steep liquor, 0.5%-3.5% of sucrose, 0.2%-0.8% of urea, 0.1%-1% of yeast extract, 0.2%-1.0% of potassium dihydrogen phosphate, and 0.02%-0.10% of a defoamer.

In some embodiments, the fermentation substrate is added before, simultaneously with, or after inoculating the seed liquid of the fermentation strain into the fermentation tank.

In some embodiments, the fermentation substrate is added or supplemented in a manner of one-time addition, batch supplementation, or continuous feeding.

In some embodiments, the seed liquid of the fermentation strain is first inoculated into a fermentation tank containing a fermentation culture medium and cultured. When the fermentation strain in the fermentation system has an optical density value OD₆₂₀ of 0.5-1.0 after a 30-fold dilution, a fermentation substrate is added to start the fermentation.

In some embodiments, during the fermentation, the viscosity in the fermentation system is maintained at an appropriate level by supplementing the fermentation substrate into the fermentation system, so that the strain maintains relatively high activity and acid-producing capacity, and a good mass transfer effect is provided between multiple phases of the fermentation system.

In some embodiments, within T hours after the start of the fermentation, a viscosity **η** and a fermentation time t of the fermentation system satisfy the following numerical relationship: **η = a × e^{bt} + c**, wherein 135 ≤ T ≤ 165, 1.05 ≤ a ≤ 5.8, 0.01 ≤ b ≤ 0.045, and 0 ≤ c ≤ 5.5; the unit of the viscosity **η** is mpa·s, and the unit of the fermentation time **t** is h. e is a natural constant, which is an infinite non-repeating decimal and has a value of about 2.718281828459045.

In some embodiments, the numerical range of T satisfies 145 ≤ T ≤ 155.

In some embodiments, the numerical range of a satisfies 1.2 ≤ a ≤ 4.5.

In some embodiments, the numerical range of b satisfies 0.012 ≤ b ≤ 0.037.

In some embodiments, the numerical range of c satisfies 0 ≤ c ≤ 3.

Those skilled in the art will appreciate that the fermentation time t is in the range of greater than 0 and less than or equal to T.

In some embodiments, the fermentation substrate is fed into the fermentation system within T hours after the start of the fermentation.

In some embodiments, the supplementation of the fermentation substrate is stopped after 135-165 h of fermentation.

In some embodiments, the supplementation of the fermentation substrate is stopped after 145-155 h of fermentation.

The supplementation of the fermentation substrate is stopped after the fermentation is performed for a certain period of time, and the fermentation is performed by utilizing the residual fermentation substrate in the fermentation system until the fermentation substrate is completely consumed or the long-chain dicarboxylic acid is no longer produced. The total fermentation time is calculated.

In some embodiments, during the fermentation, the temperature is controlled at 27-33°C.

In some embodiments, during the fermentation, the aeration rate is controlled at 0.2-0.8 vvm, and/or, the pressure is controlled at 0.05-0.20 MPa, and/or, the pH value is controlled at 4.5-6.5, preferably 6.0-6.5, and/or, the amount of dissolved oxygen is controlled at 5%-50%, preferably 25%-50%.

The fermentation broth produced by fermentation can be directly used as a product, or the long-chain dicarboxylic acid in the fermentation broth is extracted as a final product.

In some embodiments, the method comprises: subjecting a fermentation broth to a purification treatment, wherein the purification treatment comprises: acidifying the fermentation broth, performing separation to obtain a solid matter, then dissolving the solid matter in an organic solvent, subjecting the mixture to crystallization and solid-liquid separation, and subjecting the separated solid to washing and drying to obtain a long-chain dicarboxylic acid product.

In some embodiments, the method comprises: subjecting a fermentation broth to a purification treatment, wherein the purification treatment comprises: acidifying the fermentation broth, performing separation to obtain a solid matter, then dissolving the solid matter in an organic solvent, subjecting the mixture to crystallization and solid-liquid separation, and subjecting the separated solid to a hot water treatment, washing, and drying to obtain a long-chain dicarboxylic acid product.

In some embodiments, the acidification is performed at a pH of 2-5, preferably 3.5-4.5. The long-chain dicarboxylic acid in the fermentation broth is crystallized through acidification.

In some embodiments, a method of the solid-liquid separation described above comprises at least one of filtration or centrifugation.

In some embodiments, a mass ratio of the solid matter to the organic solvent is 1:(3-6).

In some embodiments, the organic solvent described above comprises one or more of an acid, an alcohol, an ester, a ketone, and the like, wherein the alcohol comprises one or more of methanol, ethanol, n-butanol, and isopropanol, the acid comprises acetic acid, the ketone comprises acetone, and the ester comprises at least one of ethyl acetate and butyl acetate. In some embodiments, after the solid matter is dissolved in the organic solvent and before the crystallization is performed, a decolorization treatment is performed.

In some embodiments, an addition amount of a decolorizing agent during the decolorization treatment is 0.5%-10%, preferably 1%-5%, and further preferably 1.2%-4.8% of the mass of the long-chain dicarboxylic acid contained in the liquid to be decolorized.

In some embodiments, the decolorizing agent is activated carbon.

In some embodiments, the decolorization is performed at a temperature of 80-110°C, and the decolorization is performed at a time of 30-190 min.

In some embodiments, the hot water treatment is performed at a temperature of 70-150°C, preferably 70-135°C.

In some embodiments, the hot water treatment comprises the following step: contacting the separated solid with hot water to reduce an impurity content, wherein a temperature of the hot water is 70-150°C, preferably 70-135°C.

In some embodiments, the hot water treatment comprises the following steps: mixing the solid with water in a mass ratio of 1:(1-10), incubating at 70-150°C for 30-180 min, cooling to 25-60°C, and performing solid-liquid separation to obtain a solid.

In some embodiments, the hot water treatment comprises the following steps: mixing the solid with water in a mass ratio of 1:(1-10), incubating at 70-135°C for 30-180 min, cooling to 25-60°C, and performing solid-liquid separation to obtain a solid.

In some embodiments, the solid obtained after the hot water treatment is washed and dried.

In some embodiments, the crystallization is cooling crystallization, and an endpoint temperature of the cooling crystallization is 20-40°C.

In a second aspect, the present disclosure is to provide a long-chain dicarboxylic acid product with a purity of 98% or higher, preferably 99% or higher.

A bio-based content of the long-chain dicarboxylic acid product can be adjusted according to different fermentation substrates, and is 1%-100%, preferably 5%-100%, more preferably 5%-99%, further preferably 5%-80%, and even further preferably 5%-50%, such as 95%, 80%, 70%, 50%, 45%, 35%, 30%, 25%, 15%, or 10%.

The present disclosure uses eco-friendly raw materials such as fatty acids, fatty acid salts, fatty acid esters, and plant-based alkanes as substrates to partially or completely replace fossil fuels such as petroleum alkanes, and the target product, i.e., the long-chain dicarboxylic acid, can still maintain relatively high yield and conversion rate.

### DETAILED DESCRIPTION OF THE INVENTION

To make the objectives, technical solutions, and advantages of the examples of the present disclosure clearer, the technical solutions in the examples of the present disclosure will be clearly and completely described below. Apparently, the described examples are only a part rather than all of the examples of the present disclosure. All other examples obtained by those of ordinary skill in the art, based on the examples in the present disclosure, without making creative efforts, shall fall within the protection scope of the present disclosure.

In the following examples, the contents of dodecanedioic acid and hexadecanedioic acid in the fermentation broth are determined by gas chromatography. Purity detection of the long-chain dicarboxylic acid product: gas chromatography. Bio-based content detection: standard ASTM-D6866 method of the American Society for Testing and Materials.

Unless otherwise specified, the percentages used to characterize component contents in the culture medium of the present disclosure all refer to general conventions in the field of fermentation, and the percentages represent weight-to-volume ratios (w/v), i.e., % represents g/100 mL.

Strain used in the examples: *Candida viswanathii* CAES2113, which has been disclosed in CN111748480A, was biologically deposited on Feb. 24, 2020, at the China Center for Type Culture Collection (address: Wuhan University, Wuhan, China), with an accession number of CCTCC M 2020048, and a taxonomic name of *Candida viswanathii*.

In Examples 1-3, n-dodecane is derived from petroleum, i.e., a petroleum-based alkane, and has a purity of 99.92%. In Example 4, n-dodecane is obtained through vegetable oil processing, i.e., a bio-based alkane, and has a purity of 99.91%. In Example 5 and Comparative Example 2, n-hexadecane is derived from petroleum, i.e., a petroleum-based alkane, and has a purity of 99.94%.

The viscosity η of the fermentation system is monitored through an online viscosity detector, and a supplementation amount of the substrate is controlled such that a numerical relationship between the viscosity **η** and the fermentation time t of the fermentation system satisfies η = a × e^{bt} + c.

### Example 1

(1) First, the pH value of a wort of 10°Bé was adjusted to about 5.4, and then 100 mL of the wort was placed in a conical flask and sterilized at 121°C for 20 min. Subsequently, 1 tube of seeds of *Candida viswanathii* (strain CAES2113) preserved in a 2 mL glycerol tube stored in a -80°C refrigerator in advance was inoculated, and activation culture was performed on a rotary shaker at a temperature of 29°C and a rotation speed of 220 rpm for 40 h. When the OD₆₂₀ value of the culture reached 0.7 (30-fold dilution), the activation culture was terminated.
(2) The shake flask seeds obtained in step (1) described above were inoculated into a seed tank containing a seed culture medium at an inoculation amount of 1.6% (v/v) and cultured, and during the culture, the temperature was controlled at 29°C, the aeration rate was controlled at 0.33 vvm, the pressure was controlled at 0.11 MPa, and the amount of dissolved oxygen was maintained at 15%, until the OD₆₂₀ value of the obtained seed liquid reached 0.8 after a 30-fold dilution.
   Components of the seed culture medium were: 0.5% of corn steep liquor (a total nitrogen content of 2.5%), 2.3% of sucrose, 0.3% of urea, 0.5% of yeast extract, 0.6% of potassium dihydrogen phosphate, and 0.05% of a defoamer.
(3) The seed liquid obtained in step (2) described above was inoculated into a fermentation tank containing a fermentation culture medium to perform fermentation, with an inoculation amount of 15% (v/v), a fermentation temperature of 29°C, a pH value of 6.4, an aeration rate of 0.31 vvm, a pressure during the fermentation of 0.12 MPa, and an amount of dissolved oxygen during the fermentation of 42%.

Components of the fermentation culture medium in the fermentation tank were: 0.9% of corn steep liquor, 3.3% of glucose, 0.4% of yeast extract, 0.5% of potassium dihydrogen phosphate, 0.2% of magnesium sulfate, 0.4% of ammonium sulfate, and 0.4% of potassium nitrate.

After the seed liquid was inoculated into the fermentation tank, when the OD₆₂₀ value of the culture reached 0.80 (30-fold dilution), feeding of substrates into the fermentation tank was started to perform fermentation. From the start of fermentation to 150 h of fermentation (i.e., T is 150 h), the viscosity **η** (unit: mpa·s) and the fermentation time t (unit: h) of the fermentation system satisfied the following numerical relationship: **η** = 2.95 × e^{0.026t} + 1.2. After 150 h of fermentation, the feeding of substrates was stopped. The fermentation substrates were lauric acid and n-dodecane in a mass ratio of 12:18.

### Example 2

Step (1): Same as that in Example 1.

Step (2): Same as that in Example 1.

Step (3): When the OD₆₂₀ value of the culture reached 0.75 (30-fold dilution), feeding of substrates into the fermentation tank was started to perform fermentation. From the start of fermentation to 152 h of fermentation (i.e., T is 152 h), the viscosity **η** (unit: mpa·s) and the fermentation time t (unit: h) of the fermentation system satisfied the following numerical relationship: **η** = 3.05 × e^{0.015t} + 0.91. After 152 h of fermentation, the feeding of substrates was stopped. The fermentation substrates were lauric acid and n-dodecane in a mass ratio of 10:20. The remaining procedures were the same as those in Example 1.

### Example 3

Step (1): Same as that in Example 1.

Step (2): Same as that in Example 1.

Step (3): When the OD₆₂₀ value of the culture reached 0.84 (30-fold dilution), feeding of substrates into the fermentation tank was started to perform fermentation. From the start of fermentation to 154 h of fermentation (i.e., T is 154 h), the viscosity **η** (unit: mpa·s) and the fermentation time t (unit: h) of the fermentation system satisfied the following numerical relationship: **η** = 4.02 × e^{0.032t} + 0.02. After 154 h of fermentation, the feeding of substrates was stopped. The fermentation substrates were lauric acid and n-dodecane in a mass ratio of 8:22. The remaining procedures were the same as those in Example 1.

### Example 4

Step (1): Same as that in Example 1.

Step (2): Same as that in Example 1.

Step (3): After the seed liquid was inoculated into the fermentation tank, when the OD₆₂₀ value of the culture reached 0.82 (30-fold dilution), feeding of substrates into the fermentation tank was started to perform fermentation. From the start of fermentation to 155 h of fermentation (i.e., T is 155 h), the viscosity η (unit: mpa·s) and the fermentation time t (unit: h) of the fermentation system satisfied the following numerical relationship: **η** = 2.98 × e^{0.022t} + 0.7. After 155 h of fermentation, the feeding of substrates was stopped. The remaining procedures were the same as those in Example 1.

### Comparative Example 1

Step (1): Same as that in Example 1.

Step (2): Same as that in Example 1.

Step (3): After the seed liquid was inoculated into the fermentation tank, when the OD₆₂₀ value of the culture reached 0.80 (30-fold dilution), feeding of substrates into the fermentation tank was started to perform fermentation. From the start of fermentation to 150 h of fermentation, the viscosity **η** (unit: mpa·s) and the fermentation time t (unit: h) of the fermentation system satisfied the following numerical relationship: **η** = 2.88 × e^{0.075t} + 0.9. After 150 h of fermentation (i.e., T is 150 h), the feeding of substrates was stopped. The fermentation substrates were lauric acid and n-dodecane in a mass ratio of 15:15. The remaining procedures were the same as those in Example 1.

The results of an acid yield, a conversion rate, and a total fermentation time of Examples 1-4 and Comparative Example 1 after the fermentation was ended are shown in Table 1.

**Table 1**

| | Acid yield/(g/L) | Conversion rate/% | Total fermentation time/h |
|---|---|---|---|
| Example 1 | 178.8 | 97.2 | 162 |
| Example 2 | 180.5 | 97.8 | 160 |
| Example 3 | 184.7 | 98.5 | 158 |
| Example 4 | 173.6 | 96.4 | 163 |
| Comparative Example 1 | 172.6 | 93.2 | 173 |

Separation and purification were performed on fermentation broths obtained in the above examples and comparative example, specifically as follows:
Sulfuric acid was added to the fermentation broth to adjust the pH to 3.5 to perform acidification crystallization, and separation was performed to obtain a solid matter, thus obtaining a crude product of dodecanedioic acid.

The crude product of dodecanedioic acid was placed into a decolorization tank. Acetic acid was added, and the mass ratio of the crude product of dodecanedioic acid to the acetic acid was controlled at 1:3. Activated carbon was added, and the addition amount of the activated carbon was 3.5% of the mass of dodecanedioic acid in the liquid to be decolorized. The above materials were heated to 92°C and decolorized for 40 min, and a clear liquid was obtained through filtration by a plate and frame filter press. The clear liquid was cooled to 30°C, and after crystals were precipitated, solid-liquid separation was performed by a centrifuge. Water was added to the obtained solid, and the mass ratio of the solid to the water was 1:10. After 90 min of incubation at 105°C, the system was cooled to 32°C, and filtration was performed to obtain a solid. The solid was washed and dried to obtain a dodecanedioic acid product. The tested purity and bio-based content of the dodecanedioic acid product are shown in Table 2.

**Table 2**

| | Purity/% | Bio-based content/% |
|---|---|---|
| Example 1 | 99.88 | 34.24 |
| Example 2 | 99.85 | 28.38 |
| Example 3 | 99.91 | 22.57 |
| Example 4 | 99.82 | 99.55 |
| Comparative Example 1 | 99.25 | 30.73 |

### Example 5

(1) Same as that in Example 1.
(2) The shake flask seeds obtained in step (1) described above were inoculated into a seed tank containing a seed culture medium at an inoculation amount of 1.6% (v/v) and cultured, and during the culture, the temperature was controlled at 29°C, the aeration rate was controlled at 0.33 vvm, the pressure was controlled at 0.11 MPa, and the amount of dissolved oxygen was maintained at 22%, until the OD₆₂₀ value of the obtained seed liquid reached 0.8 after a 30-fold dilution.
   Components of the seed culture medium were: 0.5% of corn steep liquor (a total nitrogen content of 2.5%), 1.9% of sucrose, 0.3% of urea, 0.8% of yeast extract, 0.6% of potassium dihydrogen phosphate, and 0.05% of a defoamer.
(3) The seed liquid obtained in step (2) described above was inoculated into a fermentation tank containing a fermentation culture medium to perform fermentation, with an inoculation amount of 15% (v/v), a fermentation temperature of 29°C, a pH value of 6.3, an aeration rate of 0.33 vvm, a pressure during the fermentation of 0.15 MPa, and an amount of dissolved oxygen during the fermentation of 46%.

Components of the fermentation culture medium in the fermentation tank were: 0.6% of corn steep liquor, 3.7% of glucose, 0.3% of yeast extract, 0.8% of potassium dihydrogen phosphate, 0.1% of magnesium sulfate, 0.4% of ammonium sulfate, and 0.7% of potassium nitrate.

The seed liquid was inoculated into the fermentation tank and cultured, and when the OD₆₂₀ value of the culture reached 0.77 (30-fold dilution), feeding of substrates into the fermentation tank was started to perform fermentation. From the start of fermentation to 143 h of fermentation (i.e., T is 143 h), the viscosity **η** (unit: mpa·s) and the fermentation time t (unit: h) of the fermentation system satisfied the following numerical relationship: η = 2.90 × e^{0.041t} + 1.9. After 143 h of fermentation, the feeding of substrates was stopped. The fermentation substrates were palmitic acid and n-hexadecane in a mass ratio of 1.5:15.

### Comparative Example 2

Step (1): Same as that in Example 5.

Step (2): Same as that in Example 5.

Step (3): After the seed liquid was inoculated into the fermentation tank, when the OD₆₂₀ value of the culture reached 0.77 (30-fold dilution), feeding of substrates into the fermentation tank was started to perform fermentation. From the start of fermentation to 143 h of fermentation (i.e., T is 143 h), the viscosity **η** (unit: mpa·s) and the fermentation time t (unit: h) of the fermentation system satisfied the following numerical relationship: η = 2.90 × e^{0.077t} + 1.9. After 143 h of fermentation, the feeding of substrates was stopped. The fermentation substrates were palmitic acid and n-hexadecane in a mass ratio of 1.5:15. The remaining procedures were the same as those in Example 5.

The results of an acid yield, a conversion rate, and a total fermentation time of Example 5 and Comparative Example 2 after the fermentation was ended are shown in Table 3.

**Table 3**

| | Acid yield/(g/L) | Conversion rate/% | Total fermentation time/h |
|---|---|---|---|
| Example 5 | 154.8 | 84.2 | 153 |
| Comparative Example 2 | 140.1 | 77.6 | 166 |

Finally, it should be noted that: the above examples are only used to illustrate the technical solutions of the present disclosure, rather than to limit them; although the present disclosure has been described in detail with reference to the foregoing examples, those of ordinary skill in the art should understand that: modifications can still be made to the technical solutions recorded in the foregoing examples, or equivalent replacements can be made to some or all of the technical features therein; and these modifications or replacements do not make the essence of corresponding technical solutions depart from the scope of the technical solutions of the examples of the present disclosure.

## Claims

1. A method for producing a long-chain dicarboxylic acid, comprising: inoculating a seed liquid of a fermentation strain into a fermentation tank, where a fermentation substrate comprises any one or more of a fatty acid and a derivative thereof, and an alkane.

2. The method according to claim 1, wherein the long-chain dicarboxylic acid has a chemical formula of HOOC(CH₂)nCOOH, wherein n ≥ 8; the long-chain dicarboxylic acid comprises: any one of decanedioic acid, undecanedioic acid, dodecanedioic acid, tridecanedioic acid, tetradecanedioic acid, pentadecanedioic acid, hexadecanedioic acid, heptadecanedioic acid, and octadecanedioic acid.

3. The method according to claim 1, wherein the fermentation substrate comprises any one or a combination of two or more of a fatty acid, a fatty acid ester, a fatty acid salt, and an alkane.

4. The method according to claim 1, wherein within T hours after the start of the fermentation, a viscosity **η** and a fermentation time t of the fermentation system satisfy the following numerical relationship: **η = a × e^{bt} + c**, wherein 135 ≤ T ≤ 165, 1.05 ≤ a ≤ 5.8, 0.01 ≤ b ≤ 0.045, and 0 ≤ c ≤ 5.5; the unit of the viscosity **η** is mpa·s, and the unit of the fermentation time **t** is h.

5. The method according to claim 4, wherein 145 ≤ T ≤ 155.

6. The method according to claim 4, wherein the fermentation substrate is fed into the fermentation system within T hours after the start of the fermentation.

7. The method according to claim 1, wherein the seed liquid has an optical density value OD₆₂₀ of 0.5-1.0 after a 30-fold dilution; and/or,
the seed liquid is inoculated into a fermentation tank at an inoculation amount of 10%-50%.

8. The method according to claim 1 or 4, wherein during the fermentation, an aeration rate is 0.2-0.8 vvm, and/or, a pressure is 0.05-0.20 MPa, and/or, a pH value is 4.5-6.5, and/or, an amount of dissolved oxygen is 5%-50%.

9. The method according to claim 1, wherein the seed liquid of the fermentation strain is inoculated into a fermentation tank containing a fermentation culture medium and cultured, and when the fermentation strain in the fermentation system has an optical density value OD₆₂₀ of 0.5-1.0 after a 30-fold dilution, a fermentation substrate is added to start the fermentation.

10. The method according to any one of claims 1, 3 and 6, wherein the fermentation substrate is a fatty acid and an alkane; the fatty acid and the alkane are in a mass ratio of (0.1-25):15, preferably (1-20):15.

11. The method according to claim 1, wherein a fermentation culture medium comprises the following components: 0%-1.0% of corn steep liquor, 2.0%-6.0% of glucose, 0%-0.8% of yeast extract, 0.4%-1.0% of potassium dihydrogen phosphate, 0.1%-0.6% of magnesium sulfate, 0.1%-1% of ammonium sulfate, and 0.2%-0.8% of potassium nitrate.

12. The method according to any one of claims 1, 3 and 6, wherein the alkane is a petroleum-based alkane and/or a bio-based alkane.

13. The method according to any one of claims 1-12, comprising: subjecting a fermentation broth to a purification treatment, wherein the purification treatment comprises: acidifying the fermentation broth, performing separation to obtain a solid matter, then dissolving the solid matter in an organic solvent, subjecting the mixture to crystallization and solid-liquid separation, and subjecting the separated solid to washing and drying to obtain a long-chain dicarboxylic acid product.

14. The method according to any one of claims 1-13, comprising: subjecting a fermentation broth to a purification treatment, wherein the purification treatment comprises: acidifying the fermentation broth, performing separation to obtain a solid matter, then dissolving the solid matter in an organic solvent, subjecting the mixture to crystallization and solid-liquid separation, and subjecting the separated solid to a hot water treatment, washing, and drying to obtain a long-chain dicarboxylic acid product.

15. The method according to claim 14, wherein the hot water treatment is performed at a temperature of 70-150°C.

16. The method according to claim 14 or 15, wherein the hot water treatment comprises the following steps: mixing the solid with water in a mass ratio of 1:(1-10), incubating at 70-150°C for 30-180 min, cooling to 25-60°C, and performing solid-liquid separation to obtain a solid.

17. A long-chain dicarboxylic acid product, wherein the long-chain dicarboxylic acid product has a purity of 98% or higher, preferably 99% or higher; and/or,
the long-chain dicarboxylic acid product has a bio-based content in a range of 1%-100%, preferably 5%-100%, and further preferably 5%-99%, such as 95%, 80%, 70%, 50%, 45%, 35%, 30%, 25%, 15%, or 10%.
